Europäisches Patentamt

(19) **European Patent Office**

**Office européen des brevets**

(11) Publication number: **0 162 819**
**A1**

(12) **EUROPEAN PATENT APPLICATION**

(21) Application number: **85850042.4**

(22) Date of filing: **06.02.85**

(51) Int. Cl.⁴: **C 12 N 1/02**
**B 03 C 1/00, C 12 Q 1/24**
**//G01N33/553**

(30) Priority: **01.03.84 SE 8401125**

(43) Date of publication of application:
**27.11.85 Bulletin 85/48**

(84) Designated Contracting States:
**AT BE CH DE FR GB IT LI LU NL SE**

(71) Applicant: **Sjögren, Hans-Olof**
**Skolmästarvägen 29**
**S-223 67 Lund(SE)**

(71) Applicant: **Schröder, Ulf**
**Ferievägen 9**
**S-223 67 Lund(SE)**

(71) Applicant: **Pero, Ronald**
**Klostergatan 5b**
**S-222 22 Lund(SE)**

(71) Applicant: **Miller, Daniel G.**
**6 Fox Meadow Road**
**Scarsdale New York 10583(US)**

(72) Inventor: **Schröder, Ulf**
**Ferievägen 9**
**S-223 67 Lund(SE)**

(72) Inventor: **Sjögren, Hans-Olof**
**Skolmästarvägen 29**
**S-223 67 Lund(SE)**

(72) Inventor: **Pero, Ronald**
**Klostergatan 5b**
**S-222 22 Lund(SE)**

(72) Inventor: **Miller, Daniel G.**
**6 Fox Meadow Road**
**Scarsdale New York 10583(US)**

(74) Representative: **Wiklund, Erik et al,**
**AWAPATENT AB Box 5117**
**S-200 71 Malmö(SE)**

(54) **A method for in vitro diagnosis and a diagnostic agent.**

(57) A method for in vitro diagnosis of diseases or predisposition to certain diseases in animals, including humans, is disclosed, as is a diagnostic agent for use in this method. For the diagnosis, a suspension of viable cells from animals is contacted with magnetic spheres, which spheres are adsorbed on the cell walls or incorporated in the cells. After that, the magnetised viable cells are separated from the suspension by application of an external magnetic field. The viable cells are then analysed with regard to cell parameters, such as DNA repair proficiency and/or enzyme activity and/or immunological reactivity.

The diagnostic agent consists of magnetic spheres comprising magnetic particles enclosed in a polymer matrix.

# A METHOD FOR IN VITRO DIAGNOSIS AND A DIAGNOSTIC AGENT

The present invention comprises a method for in vitro diagnosis of diseases or predisposition to diseases in animals, including humans, and a diagnostic agent for carrying the method into effect.

By analysing certain parameters in cells from sick animals, including humans, or from humans or animals predisposed to certain diseases, it is possible to diagnose the disease and also to determine how serious it is, whereby suitable treatment can be begun.

Initially, only certain parameters could be determined on nonviable cells that had been isolated from their original environment. From these parameters, it was then tried to draw specific conclusions as to the type of the disease and its seriousness, but the uncertainty was great. Thus, one cannot know for certain that what is applicable to a nonviable cell is also applicable to the viable cell in the human or the animal.

Recent methods for incubating viable cells have opened new prospects of analysing cell activities in normal cell environment. However, to be able to make such analyses, cells of a given type must be isolated from cell mixtures in different culture media. The cell composition can be determined by microscopy or by means of complicated and expensive instruments (for example FACS, i.e. fluorescein activated cell sorter). To analyse different functions in the cells, it is necessary first to separate the different cell types from one another. In this manner, interaction between different cell types, i.e. that the presence of one cell type affects the function of other cell types, is avoided.

The separation methods known so far include multistage separation in which about 10-20% of the cells are lost in each stage.

These known methods frequently necessitate the use of expensive equipment of the type FACS cell sorter and considerable manual skill of the laboratory staff to be able to achieve reproducibility.

The present invention provides a novel method of quickly and accurately separating viable cells of a given type from a cell culture or blood. This method requires no expensive equipment and can be readily carried out on a large scale and with high reproducibility also by less skilled laboratory workers.

The invention thus comprises a method for in vitro diagnosis of diseases or predisposition to certain diseases in animals, including humans, said method being characterised in that a suspension of viable cells from animals is contacted with magnetic spheres, which spheres are adsorbed on the cell walls or incorporated in the cells, and that the viable cells thus magnetised are separated from the suspension by application of an external magnetic field, whereupon the viable cells are used for analysis of cell parameters, such as DNA repair proficiency and/or enzyme activity and/or immunological reactivity.

The invention also comprises a diagnostic agent for in vitro diagnosis of diseases or predisposition to certain diseases in animals, including humans, said agent being characterised in that it consists of magnetic spheres comprising magnetic particles enclosed within a polymer matrix.

Magnetic spheres preferably are micro- or nanospheres or -particles, the average diameter of which lies within the micro- or nanometer range and which can be activated by a magnetic field.

Suitable magnetic materials for use in the nanospheres are described and generally available, for

example $Fe_3O_4$, Ni-NiO, Ni-TiO$_4$, Mn-Zn, ferrite, Ni etc. Preferably, use is made of magnetite ($Fe_3O_4$) because it is readily available and does not oxidise in aqueous environment.

The magnetic material is enclosed in a polymer material, for example acrylic derivatives, agarose, proteins, copolymers of acryl and agarose, starch and cellulose.

Preferably, use is made of a carbohydrate polymer as matrix because this material is readily formed into spheres of the desired size range. Examples of suitable carbohydrate polymers are dextran, pullullan, glycogen, starch, cellulose, agarose, alginate, chitosan or carrageenan. Dextran, cellulose and agarose are preferred because they are biochemically well-characterised and inert in most biological systems. The carbohydrate polymer may also be modified with, for example, acetyl, hydroxypropyl, propionyl, hydroxyethyl, hydroxypropanoyl groups, different derivatives of acrylic acid or the like.

Magnetic spheres having an average diameter in the range 50-900 nanometers may be produced by, for example, the method disclosed in Swedish patent application 8201972-0, in which method a carbohydrate polymer is dissolved in a solvent having a high dielectric constant to a concentration which normally lies within the range 0.1-150% (weight/volume). Useful solvents are, inter alia, dimethylformamide, ethylene glycol, sulpholan, dimethylsulphoxide, propylene carbonate, water and formamide, or mixtures thereof. The magnetic material is suspended in the dissolved carbohydrate polymer. The magnetic material consists of, for example, magnetite ($Fe_3O_4$) having a size of 10-20 nm.

A method of producing these small magnetite particles is already known, and the particles are commercially available from Ferrofluid Corp., USA. Other useful magnetic materials include particles or colloids having a substantial content of aluminium, nickel, cobalt, copper, silver, manganese or platinum.

Then the resulting suspension of magnetic material in the dissolved carbohydrate polymer is emulsified in an emulsion system consisting of said magnetic suspension and an emulsion medium consisting of a liquid immiscible with the said magnetic suspension and having the further property of contributing to the formation of small droplets of magnetic suspension in the emulsion medium.

Useful emulsion media are, for example, vegetable oils, preferably rapeseed or corn oil. Other useful hydrophobic emulsion media include paraffin oils or silicone oils. Another type of emulsion medium includes organic solvents in which one or more emulsifiers have been dissolved. Useful such organic solvents include, inter alia, xylene, toluene, ethyl benzene, diethyl benzene, propyl benzene, ethylene chloride and other similar solvents, and mixtures thereof.

To emulsify the emulsion, use is made of a sonicator or high-pressure homogeniser. The resulting emulsion in which the carbohydrate magnetite suspension is emulsified in the form of droplets, is stabilised by transferring it to a liquid capable of crystallising the carbohydrate polymer, whereby the magnetic material is enclosed in the polymer. Useful such liquids are ethanol, methanol and acetone, acetone being preferred. After crystallisation, the nanospheres are further washed with acetone, whereupon drying is effected simplest by rotational evaporation or air-drying.

To facilitate the adsorption of the magnetic spheres on cell walls, it may in some cases be necessary to use an activating substance, a so-called activator, which first is coupled to the magnetic sphere and then is bonded (preferably by covalent bonding) to the cell wall. An example of such an activator is tresyl chloride.

Individual risk estimation from genotoxic exposure depends on the isolation of viable cell populations from, for example, blood, bone marrow, skin and malignant or benign tumor tissues. Great advantages are obtained by making genotoxic risk determinations on narrowly defined cell populations, and the effect of alterations in the composition of the total population is clearly distinguishable from dissimilarities in the characteristics of a specific cell subpopulation.

The individual risk of being affected by specific diseases can be controlled by genetic or environmental factors.

Expression of both of these factors is dependent upon quantification on viable cells since physiological processes can control either

(a)   the metabolic activation and degradation of carcinogens/mutagens;

(b)   pools of precursors, co-factors or endogenic co-substrates; or

(c)   the measure of the DNA damage caused.

(1)   Genetic factors

Genetic control of individual variations in response to toxic environmental exposure has been defined as ecogenetics (Vogel, F., Buselmaier, W., Reichert, W., Kellermann, G., Berg, P. (eds) Human genetic variation in response to medical and environmental agents: Pharmacogenetics and ecogenetics. Human Genetic Suppl. 1:1-192 (1978)). Examples of ecogenetic factors capable of controlling the risk of genotoxic exposure are as follows:

(a)   Microsomal mixed-function oxygenases

Most carcinogens/mutagens require oxidative metabolism via the formation of DNA damaging epoxides before they become active as carcinogens/mutagens. Thus, the amounts of enzyme and co-factors, such as NADPH, participate in the control of how much DNA damage can be induced. Since microsomal enzymes are enclosed in certain cell compartments where they are membrane-bound, it is highly important that such determinations be made on viable cells in order to make individual risk estimations based on genetic mechanisms of predisposition.

(b) Drug metabolising enzymes

Once potential DNA damaging epoxides have been formed, they can be inactivated to non-DNA damaging metabolites by epoxide hydrolase and glutathione transferase. Thus, the amounts of these enzymes in relation to each other and in relation to microsomal mixed-function oxygenase content as well as co-factor concentrations, such as glutathione, control the intracellular balance between DNA damaging intermediates. As a result, this intracellular balance can be controlled genetically, and again the importance of using viable cells to estimate the human risk is emphasised. Other drug metabolising enzymes which may be of importance in this respect and which can be detected in blood cells, are NADPH cytochrome P-450 reductase, NADH cytochrome C reductase and DT diaphorase.

(c) DNA repair enzymes

There is precedence that individuals may suffer from deficient ability to repair DNA damage. A deficiency of this type may lead to a genetic predisposition to cancer. Since it has not been possible so far to purify any DNA repair enzymes from human sources, and since there is a strong interdependence between the different DNA repair enzymatic activities (i.e. the activities of endonuclease, exonuclease, polymerase and ligase), analysis has so far been carried out more indirectly. Unscheduled DNA synthesis (UDS = Unscheduled DNA synthesis), DNA strand breakage, carcinogen removal, ADP ribosyl transferase activity, mutation frequency analysis, micronuclei production, chromosome aberrations, sister chromatide exchange and other cytogenetic parameters are dependent on nucleotide precursors and the enzymes producing or degrading them, nucleotide pool imbalance and cofactors, such as Mg and $NAD^+$. All of these analysis procedures require the culturing of viable cells, and some are dependent on cell division. Moreover, cellular control of the DNA repair enzymatic activity via nucleotide pool limitations is of importance in estimating the genotoxic risk, and this type of control factor can also be analysed by culturing viable cells.

(2) Environmental factors

It is now well documented that humans occupationally exposed to a variety of chemicals develop cancer and other diseases. A number of different tests have been developed to evaluate hazardous environmental exposure. Again nearly all of these procedures require isolation and culturing of viable cells. The procedures for estimating the individual genotoxic risk mentioned above have previously been developed with cell systems from animal models, and data have been extrapolated to humans. Hence it follows that the dependence of analysis procedures for estimating the risk of carcinogens/mutagens on viable cell populations also is true for other mammals, such as the rat, mouse, hamster, monkey etc.

(a) <u>Occupational and industrial exposure</u>

The most common procedure for evaluating if a chemical is hazardous to health, is to expose cells in vitro to the test chemical and to evaluate the individual genotoxic response. This cannot be done without culturing viable cells in the presence of the test chemical. The in vivo risk is often evaluated similarly, but comparison between in vitro responses is made for individuals not exposed in vivo to the test chemical.

(b) <u>Nutritional effects</u>

Peroxidative processes in the cell can produce oxygen radicals that damage DNA, and they can activate carcinogens/mutagens to DNA-binding intermediates by co-oxidative mechanisms. The DNA damage induced by these paths is quickly influenced by the presence of anticarcinogens in the intracellular chemical pool. Anticarcinogens are often radical scavengers, such as vitamin E, β-carotene, glutathione, ascorbic acid or uric acid, and the levels of these substances in the cells are controlled in part by diet. In order to analyse the individual risk, it therefore is important to carry out analyses on viable cells where the influence of these factors can be taken into consideration. Lipid nutrition is also of importance in this respect. Membrane fluidity, lipoxygenase and cyclooxygenase (prostaglandin synthetase) are controlled either by phospholipid-bound or by free arachidonic acid. The ratio of saturated to unsaturated extracellular fatty acids can, in its turn, adjust the intracellular fatty acid pool. An enriched intercellular pool of unsaturated fatty

9

acids would increase the opportunities for oxygen radical formation and for co-oxidative activation of carcinogens/mutagens. Again, appropriate risk estimation should be carried out on viable target cells only where these regulators of the genotoxic risk can be evaluated.

Furthermore, analysis of the DNA repair proficiency of viable cells has shown that

a) persons with high blood pressure;

b) elderly people;

c) persons exposed to ethylene oxide;

d) persons exposed to styrene;

e) persons having a genetic disposition to colon cancer;

f) persons who have or have had colon cancer have all had less DNA repair proficiency after short-term exposure of the cells to a carcinogen.

Similarly, it has been found that the activity of another enzyme (ATP ribose transferase) in cells from patients suffering from leukemia is directly connected with the degree of malignity of the disease. In other words, this analysis makes it possible directly to decide whether or not the patient can be cured.

Another field in which the possibility of analysing on viable cells is of great importance is the analysis of immunological reactivity. The function of different types of leucocytes frequently is aggravated by the fact that the composition of the tested cell polulations varies, that the function tests are strongly dependent on the cell number, and that different cell types affect one another, i.e. that the presence of a given cell type affects the function of other cell types. Examples of this application are the following.

1: The presence of T suppressor lymphocytes may make it impossible to demonstrate a possibly existing T helper activity or T cytotoxycity activity. Inversely, measuring the function of the T suppressor may be rendered impossible by the simultaneous presence of varying amounts of effector cells.

2: Analysis of the cytotoxic effect of cytolytic T cells can be rendered impossible if NK cells are present at the same time. Inversely, the activity of the NK cells can be assessed more accurately if T lymphocytes are not present at the same time.

Simultaneous separation of several cell subpopulations makes it possible to analyse the cell-cell interaction, for example inhibition of different T and B cell functions of graduated admixture of T suppressor cells. Another example is the controlled admixture of monocytes of a given type to lymphocytes for correct assessment of the lymphocyte reactivity, for example proliferative T cell response to exposure to antigen which does not occur in the absence of monocytes.

These types of analysis are highly important to cancer patients, patients obtaining organ transplantate, patients suffering from autoimmune diseases, and individuals having acquired immune defects (AIDS) or congenital immune defects.

The corresponding analysis of inflammatory cells present in tumor tissue is analogous to the above analysis, although complicated by the simultaneous presence of a large number of tumor cells of varying characteristics. These tumor cells must first of all be separated from the inflammatory cells, which can be done by access to monochlonal antibodies directed against different tumor markers. It is also important to study purified tumor cell suspensions with respect to different cell parameters. Analogue conditions apply also to tissue in which an autoimmune process is in progress, where inflammatory cells must be separated from fibroblasts and endotel and epithelial tissue cells.

To illustrate the invention in more detail, the following Example is given which, however, is not intended to restrict the scope of the invention.

EXAMPLE

60 mg of tresyl-activated magnetic spheres were used for coupling 1 mg of protein A, whereupon the magnetic spheres were stabilised with 1,6-diamino hexane and ethylene diamine. Antihuman IgG was then adsorbed to these protein A spheres. After careful washing of unbound ligand, these magnetic spheres were incubated with 53 million mononuclear human cells from peripheral blood in 4 ml of PBS for 1 hour at $+4^{\circ}C$.

After magnetic separation, 8 million cells were obtained which had been adsorbed and separated from the cell mixture.

On these "magnetic cells", a determination of DNA repair proficiency (UDS) according to standardised technique was then carried out, with the following result.

4 million cells per culture.

|  | cpm | µg DNA | cpm/µg DNA | UDS |
|---|---|---|---|---|
| HU | 1035 | 8.5 | 122 | |
|  | 950 | 9.2 | 103 | |
| NA-AAF | 3263 | 6.1 | 537 | |
|  | 3412 | 5.5 | 619 | 466 |

This value of UDS may be regarded as entirely normal, which shows that the magnetic separation and the fact that the cells are covered by magnetic particles, do not affect the analysis results.

12

## CLAIMS

1. A method for in vitro diagnosis of diseases or predisposition to certain diseases in animals, including humans, c h a r a c t e r i s e d in that a suspension of viable cells from animals is contacted with magnetic spheres, which spheres are adsorbed on the cell walls or incorporated in the cells, and that the viable cells thus magnetised are separated from the suspension by application of an external magnetic field, whereupon the viable cells are used for analysis of cell parameters, such as DNA repair proficiency and/or enzyme activity and/or immunological reactivity.

2. A diagnostic agent for in vitro diagnosis of diseases or predisposition to certain diseases in animals, including humans, c h a r a c t e r i s - e d in that it consists of magnetic spheres comprising magnetic particles enclosed within a polymer matrix.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | WO-A-8 303 426 (GAMBRO LUNDIA AB) * Whole document * | 1,2 | C 12 N    1/02<br>B 03 C    1/00<br>C 12 Q    1/24 //<br>G 01 N   33/553 |
| X | EP-A-0 016 552 (NORTHWESTERN UNIVERSITY) * Whole document * | 1,2 | |
| X | US-A-3 970 518 (I. GIAEVER) * Whole document * | 1,2 | |
| X | WO-A-8 200 660 (CORNING GLASS WORKS) * Whole document * | 1,2 | |
| X | NATURE, vol. 268, August 4, 1977, pages 437-438, Basingstoke, GB; R.S. MOLDAY et al.: "Application of magnetic microspheres in labelling and separation of cells" * Whole article * | 1,2 | TECHNICAL FIELDS SEARCHED (Int. Cl.4)<br><br>G 01 N<br>C 12 Q<br>C 12 N<br>B 03 C<br>B 01 D |

--- -/-

The present search report has been drawn up for all claims

| Place of search | Date of completion of the search | Examiner |
|---|---|---|
| THE HAGUE | 11-07-1985 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 89, no. 7, August 14, 1978, page 264, no. 55993u, Columbus, Ohio, US; P.L. KRONICK et al.: "Magnetic microspheres prepared by redox polymerization used in a cell separation based on gangliosides" & SCIENCE 1978, 200(4345), 1074-6 * Whole abstract * | 1,2 | |
| | ----- | | |
| | | | TECHNICAL FIELDS SEARCHED (Int. Cl 4) |

The present search report has been drawn up for all claims

| Place of search | Date of completion of the search | Examiner |
|---|---|---|
| THE HAGUE | 11-07-1985 | GRIFFITH G. |